# EUROPEAN PATENT APPLICATION

(11) **EP 4 726 024 A1**
(43) Date of publication of application: **15.04.2026**
(21) Application number: 24461626.4
(22) Date of filing: 10.10.2024
(51) Int. Cl.: C12M 1/00, C12M 1/12, C12M 1/34, C12M 1/36

(54) **BIOREACTOR, MODULAR BIOTECHNOLOGICAL INSTALLATION AND METHOD OF PRODUCTION OF GASES USING MODULAR BIOTECHNOLOGICAL INSTALLATION**

(71) Applicant: Gazda, Jacek, 43-241 Laka (PL)
(72) Inventor: Gazda, Jacek, 43-241 Laka (PL)
(74) Representative: Dominiak, Malgorzata Maria

(57) **Abstract**

The present invention relates to a bioreactor and a modular biotechnological installation for producing a gas mixture using bioorganisms and/or algal biomass and biological products resulting from their growth. The invention also relates to a method of production of gases using the modular biotechnological installation according to the invention.

## Description

### Technical field

The present invention relates to a bioreactor and a modular biotechnological installation for producing a gas mixture by using bioorganisms and/or algal biomass and for producing biological products resulting from their growth. The invention also relates to a method of production of gases by using the modular biotechnological installation according to the invention.

### Background

Global demand for energy is constantly growing. At the same time, awareness of an impact of greenhouse gases on the environment is increasing. To assess harmfulness of human activities, the concept of "carbon footprint" can be used, which refers to an amount of greenhouse gases, including carbon dioxide (CO₂), emitted into the atmosphere in a context of activities of a given enterprise, performance of activities by an individual or in connection with production of a product. In order to meet challenges facing global energy industry and in an interests of environmental protection, it is necessary to develop usage of renewable energy sources (RES). It seems that the greatest development prospects lie in production of energy from biomass and bioorganisms and the use of said biomass for other purposes.

Algae are simple, autotrophic, tissueless organisms (thalliforms), unicellular (microalgae) or multicellular (macroalgae). One of main advantages of algae is their rapid growth. These microorganisms can double their mass within 24 hours (in favorable conditions even within 3.5 hours).

Currently, production of hydrogen as an energy source is based on use of electrolysis. This technology involves excessive use of inland waters and sea and ocean waters, which are a limited and valuable natural resource. Due to distance between a location of gas production and a location of gas use, it is often necessary to transport the gas to the destination, which increases the carbon footprint of producing such an energy source.

### Definitions

For the purposes of this application, a bioreactor should be understood as a device enabling microbiological processes, enzymatic processes and the cultivation of cells of higher organisms. It allows for control of production process, measurement and regulation of parameters in conditions of a maximum limitation or a complete elimination of the possibility of contamination/infection.

For the purposes of this application, a modular biotechnological installation should be understood as a set of dedicated modular stationary technical devices technologically linked to a bioreactor. A module is a separate, distinguishable unit of the installation that can be used as its component.

Biological products should be understood as products resulting from cultivation, containing naturally occurring molecules or organisms.

Biomass means energy material derived from products, waste and plant and animal residues. It is obtained in agriculture and forestry, in households, as well as in industry. It is the most accessible raw material, that can be easily converted into an efficient source of energy. Since algae are recognized as plants (except cyanobacteria) according to international agreements, in this application algae are defined as biomass or algal biomass. The use of biomass is consistent with the assumptions of the CCU (*Carbon Capture* and *Utilization*) Technology: Carbon dioxide capture and utilization technologies allow for closing the carbon cycle in the production cycle, reducing net emissions.

Bioorganisms should be understood as:
a/ microorganisms - understood as any microbiological unit, cellular or non-cellular, including viruses and viroids, capable of replicating or transferring genetic material, including animal and plant cell cultures;
b/ genetically modified microorganisms (GMM) - understood as a microorganism in which the genetic material has been changed in a way that does not occur under natural conditions as a result of crossbreeding or natural recombination, in particular using i) nucleic acid recombination techniques, ii) direct incorporation of genetic material prepared outside the microorganism, including microinjection, macroinjection or microencapsulation, iii) cell fusion or hybridization techniques.

A feed biomass is a biomass obtained as a result of a biological production process in the bioreactor and the modular biotechnological installation and in a separate cultivation/feed module ensuring the stability of the installation's demand for carbohydrates and other nutrients necessary in the installation. Alternatively, the source of the feed biomass may be waste from other processes.

An organic medium is a substance fed to the technical/biotechnological installation, fulfilling a role appropriate to the nature of this installation. In this application, in the embodiments, the organic medium was brine containing algal biomass dispersed therein.

Used organic medium is a substance that was removed from the technical/biotechnological installation, which does not fulfill the role appropriate to the nature of this installation but can be used in another internal process or, after meeting required parameters, used in external processes or constitute a finished product.

### State of the art

In the state of the art, biotechnological installations, in particular bioreactors, are known in form of open overflow tanks (ORP) or a photobioreactor system (PBs).

A modular bioreactor for cell culture is known from document CN219449730U, comprising a plurality of hollow reactor modules which, when connected together, form a cylindrical body provided with an external support. Each of the modules, which makes the cylindrical body, has a disposable reaction bag therein. The modules include a side plate and a bottom plate, connected to a vent pipe which communicates with a pressure valve.

Document US10246724B2 discloses a modular renewable energy system comprising a solar collector for generating thermal energy, a bioreactor comprising a chamber for fermenting organic waste, and a fuel cell or a heat engine in a gas-flow communication with the bioreactor. Fermentation of organic waste in the chamber generates hydrogen, which in turn is directed to one of the fuel cell or the heat engine.

US2011136225A1 discloses a bioreactor system that includes one or more bioreactor modules that are individually controllable and identifiable. The bioreactor module may be connected to one or more functional modules, such as, for example, a pump module, a signal generation stimulation module, a motor module, a mechanical transmission module, a gas exchange module, a temperature module, a humidity module, a CO₂ module. The bioreactor system may be controlled and/or monitored by a controller that individually identifies and controls each connected module, which may be adapted to collect signal data from sensors immersed in any of the modules.

WO2009063296A2 discloses an independent, energy-sufficient biofuel production system comprising a bioreactor for producing biomass, comprising a first microorganism and at least one substrate, a fluid and nutrients. The bioreactor is functionally connected to a biodiesel reaction chamber, wherein glycerol and biofuel are produced in this chamber from the reaction of the raw material with alcohol in the presence of a base, and wherein glycerol is used as a fuel source to drive various aspects of the system. This document indicates that the biofuel, among others, may be hydrogen.

### Objective/technical problem to be solved

Open overflow tanks can have a sufficiently large capacity, but lack of a closure means that they do not provide adequate efficiency and protection against contamination. In case of standard large-capacity bioreactors, there are significant limitations in the possibility of choosing a location for placing such a bioreactor due to its large size. In addition, ORPs are dependent on atmospheric conditions at their location, because they do not have the possibility of regulating temperature inside the device. Photobioreactor systems, on the other hand, which are usually in a form of portable installations, are very small in volume, expensive to manufacture and operate. The construction of photobioreactors, due to the materials used, most often stainless steel, does not allow them to be scaled up. The above technical problems have been solved by the present invention.

### Summary of the Invention

In a first aspect, the invention relates to a closed liquid bioreactor configured to operate under pressure, which is in a form of a tank made of an elastic material with a modulus of elasticity ranging from 0.02 to 9.5 GN/m², preferably between 1.8 and 4.0 GN/m², wherein the flexible material preferably comprises polyvinyl chloride or polyester or nylon.

Preferably, the flexible material has a layered structure and comprises a polyester carrier layer, wherein on each of the two sides of the polyester carrier there are successively:
- an impregnating layer,
- a main coating, preferably comprising PVC or polyester or polypropylene,
- a binding primer layer,
- a topcoat layer.

Preferably, the tank outlet is made of a material limiting emission of gaseous products, comprising at least two matrices, wherein the material preferably comprises a main jacket and a collar, wherein the collar is made of the same or a different material than the material from which the jacket is made.

In a further aspect, the invention relates to a modular biotechnological installation for culturing microorganisms, which comprises:
- a closed liquid bioreactor adapted to operate under pressure according to the invention, and
- a pumping system configured to allow the flow of an organic medium comprising bioorganisms and/or algal biomass between the closed tank and remaining modules of the modular biotechnological installation,
- a thermostatic module configured to increase, decrease or maintain a set temperature of the organic medium in the tank,
- a sterilization module configured to sterilize the organic medium,
- a gas exchange module configured to supply oxygen and/or CO₂ to the closed tank,
- inert piping configured to connect the tank, modules and the pumping system in the installation,
- a dosing module configured to supply food/nutrients to the organic medium,
- a lighting module configured to regulate light and dark cycle for growth of the bioorganisms and/or algal biomass,
- an energy module configured to provide electrical energy for operation of the modules of the installation,
- a measurement module configured to measure at least one of the parameters selected from: energy, temperature, pressure, salinity, pH, certainty factor-CF, redox index-ORP, thermal coefficient-S.G., electrical conductivity-EC, total content of mobile charged ions-TDS, turbidity of the organic medium, amount of biomass, concentration of gases and providing measurement data to the control module, wherein the control module is configured to control the operation of the biotechnological installation.

Preferably, the modular biotechnological installation comprises a purification module configured to remove excess organic medium from the closed tank and/or a gaseous product purification module, preferably in a form of a filter system and/or a filtration-separation module for purifying air taken from the atmosphere and/or an analysis module for analyzing measurement data from the measurement module, wherein the installation preferably comprises all of the above-mentioned modules at the same time.

Preferably, the modular biotechnological installation comprises an electromagnetic radiation module configured to generate a low frequency electromagnetic field during the flow of the organic medium and/or a pressure module configured to increase or decrease pressure during the flow of the organic medium and/or an ultrasound module configured to emit ultrasounds in the organic medium and/or an electrophoresis module configured to apply voltage to the organic medium to separate different fractions of the organic medium and/or an electroporation module configured to damage cell membrane of the cells and/or a renewable energy source module and/or a feed module for culturing bioorganisms or a feed biomass intended to feed the organic medium of the closed tank and/or a separation module for bioorganisms and/or algal biomass, wherein the installation preferably comprises all of the aforementioned modules.

In a further aspect, the invention relates to a method of production of gases using a modular biotechnological installation, which comprises the following steps:
- providing a modular biotechnological installation according to the invention,
- supplying an organic medium containing bioorganisms and/or algal biomass to a closed tank and partially or completely, preferably completely, filling the closed tank with the organic medium,
- supplying a gas mixture to the closed tank,
- converting the gas mixture into gaseous products,
- returning a part of the concentrated mixture of gaseous products obtained in the previous step, containing CO₂ to the closed tank,
- supplying food for the organic medium to the closed tank,
- removing excess of used organic medium from the closed tank,
- re-supplying the organic medium to the closed tank,
wherein during performance of the method, the recovery of the gaseous product from the closed tank is carried out, and measurement of operating parameters of the installation and transfer of data to the control module is carried out.

Preferably, the steps of the method are carried out continuously or periodically depending on the conditions in the biotechnological installation measured by means of the measurement module.

Preferably, the gas mixture in the step of supplying the gas mixture to the closed tank is a mixture containing CO₂. CO₂ can come from, for example, air, air and a CO₂ cylinder, it can also be formed as a result of growth of the organic medium, it can come from the feed module for the feed biomass cultivation, and also from a supplied cylinder containing CO₂. Alternatively, CO₂ can come from external supplies from other processes to ensure a circular economy.

Preferably, the gas product is a mixture containing hydrogen, nitrogen, oxygen and CO₂, which is then separated into fractions of individual gases.

Preferably, the step of supplying food for the organic medium takes place via the feed module for the feed biomass cultivation, whereby the entire installation becomes self-sufficient and does not require external supply of food for the organic medium.

Preferably, the modular biotechnological installation according to the invention is used to carry out the method, wherein at least one module is operated periodically during the performance of the method.

Alternatively, the modular biotechnological installation according to the invention is used to carry out the method, wherein at least one module operates continuously during the performance of the method.

The material from which the tank of the closed liquid bioreactor is made does not contain fluorides and may be, for example, polyvinyl chloride, polyester, nylon. Such a design minimizes the use of compounds belonging to the group of so-called forever chemicals in the device, including per- and polyfluoroalkyl compounds and their derivatives, which are harmful to health and their presence in the environment is permanent. Therefore, the material from which the closed liquid bioreactor is made responds to the urgent need to eliminate such per- and polyfluoroalkyl compounds from the environment.

The measurement module, the analysis module and the control module for controlling the operation of the biotechnological installation provide the possibility of changing the operating parameters in the bioreactor and the operating parameters of the remaining modules, if any, ensuring the most optimal conditions for the growth of microorganisms in the bioreactor and the production of the gas mixture.

The purification module is used to purify the organic medium from old microorganisms, which are removed from the bioreactor, for example, using a brush, automatic robots or by sucking them out of the bottom of the bioreactor. The purification module can additionally store old organisms for further internal use as food or as a product for external use.

The thermostatic module provides the ability to increase or decrease the temperature depending on external temperature to maintain a constant optimal temperature inside the closed liquid bioreactor.

The sterilization module, which allows the organic medium to be maintained in optimal condition, neutralize contaminants that appear there, for example in a form of pathogenic bacteria.

The lighting module is included in the modular biotechnological installation to maintain optimal conditions of the light and dark cycle for the growth of the organic medium.

In the gaseous product purification module, some of the products are returned to the closed liquid bioreactor, and some become the final product and are intended for further use. This reduces the amount of CO₂ emitted to atmosphere, and therefore the carbon footprint of gas production using the modular biotechnological installation according to the invention is reduced.

The electromagnetic radiation module allows for the introduction of a low-frequency electromagnetic field into the closed liquid bioreactor, which has a positive effect on microorganisms, causing acceleration of enzymatic processes and faster regeneration of slightly damaged cells and elimination of the weakest cells.

The pressure module, through the use of increased pressure and negative pressure, improves the gas exchange of microorganisms cultivated in the biotechnological installation, increases the work efficiency of the algal biomass. In case of application of an overpressure, the gas exchange with the algal biomass increases, while in the case of use of a negative pressure, the microorganisms are stimulated to increase the production of the gas mixture.

The ultrasound module stimulates the work of microorganisms. The electrophoresis module, by applying voltage, stimulates microorganisms to produce the gas mixture with increased efficiency. The electroporation module allows for increasing the efficiency of gas exchange of microorganisms located in the closed liquid bioreactor and stimulating them to grow and produce gases. The energy module, which can come from renewable energy sources, allows for reducing the carbon footprint associated with the production of the gas mixture. The filtration-separation module is used to purify the air taken from the atmosphere.

The feed module, also known as the breeding module, is used to breed bioorganisms or feed biomass intended to feed the organic medium of a closed liquid bioreactor. Feed biomass is biomass grown in the feed module, e.g. algae, whose growth products or the biomass itself can be an addition to the food for the actual biomass. The feed biomass may additionally contain plant material containing cellulose as a nutrient for algae.

The aforementioned feed module ensures self-sufficiency and no need to supply food for the organic medium from outside. This solution also means that the entire biotechnological installation reduces its carbon footprint, because the organic medium used can also be food for the feed biomass, and the CO₂ emitted by the organic medium can be introduced into the feed module, where it is consumed by the feed biomass. The feed module also draws air, including CO₂ from the atmosphere, wherein CO₂ is then conducted from the feed medium to a closed liquid bioreactor for use by the organic medium. Thanks to this solution, it is possible to create a closed circuit of the entire biotechnological installation, as well as to reduce the carbon footprint of the operating modular biotechnological installation.

The separation module for bioorganisms and/or algal biomass is advantageously configured for the separation of dead and alive bioorganisms and/or dead and alive algal biomass. The operation of the separation module for bioorganisms and/or algal biomass, also referred to as the excess bioorganisms and/or algal biomass extraction module, is advantageously based on the differences in buoyancy and flotation of dead and alive bioorganisms and/or dead and alive algal biomass.

In addition, the separation module for bioorganisms and/or algal biomass may be configured to:
- remove dead bioorganisms and/or dead algal biomass from the installation and/or
- remove excess alive bioorganisms and/or excess alive algal biomass from the installation. Excess alive bioorganisms/alive algal biomass may be transferred to another installation.

The advantage of the present invention is the possibility of easy assembly of the biotechnological installation by using a closed liquid bioreactor, which is adapted to operate under pressure and made of flexible material. The costs of building a modular installation according to the invention and the carbon footprint of building the installation compared to currently used installations are much lower, and the construction itself is quick due to the presence of a bioreactor made of flexible material. Flexible material is much cheaper than the currently most commonly used stainless steel and allows the bioreactor to be set up in a way that allows it to be easily moved from one place to another. In addition, the use of flexible materials free of chemical compounds from the group of so-called forever chemicals is environmentally friendly and meets the assumptions of planned legal regulations aimed at minimizing the use of these compounds in all possible sectors of industry. The construction of a modular biotechnological installation also allows for easy adjustment of the desired parameters and connection of individual modules to existing installations without a need for costly adaptations. The modules of the modular biotechnological installation according to the invention can be configured in any way that will ensure the operation of the installation.

In addition, due to the use of flexible material for the construction of a closed liquid bioreactor of a modular biotechnological installation, it is possible to significantly increase the scale of production of the gas mixture in a modular biotechnological installation with a volume of the closed liquid bioreactor of up to 1000 m³. Applying the appropriate pressure during the operation of the biotechnological installation allows for an increase in the efficiency of gas production.

Another advantage of the present invention is the inclusion of a data analysis module and a control module for controlling the operation of the biotechnological installation in such a way as to maintain the highest possible efficiency of the gas mixture production, but also to turn individual modules on and off in such a way as to ensure a reduction in the carbon footprint of the operating modular biotechnological installation, as well as a state close to self-sufficiency in terms of energy obtained from food for the organic medium.

Hydrogen produced in the installation according to the invention does not use limited resources of drinking water, which is in line with global economic trends such as the Sustainable Development Goals (SDGs) - Agenda 2030. The biomass produced (multiplied) in the installation can be a low-emission food for people and animals or can be transformed into biofuels, cosmetics, bioplastics, agricultural products, fertilizers or used as a storage for captured CO₂.

### Description of Figures

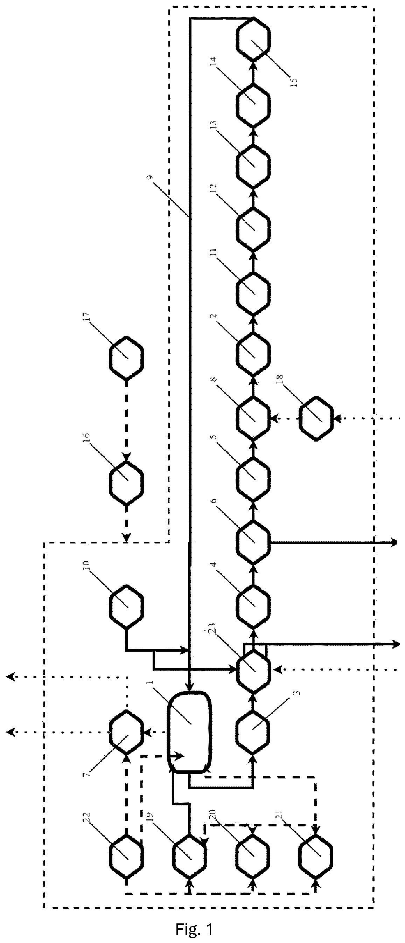

Figure 1 shows a schematic diagram of construction of a modular biotechnological installation according to Example I, comprising a closed tank 1, a pumping system 3, a measurement module 22, a feed module 19 for culturing feed biomass, a product purification module 7, a lighting module 15, a dosing module 10, a separation module for algal biomass 23, a purification module 4, a sterilization module 6, a thermostatic module 5, a gas exchange module 8, a filtration-separation module 18, an electromagnetic radiation module 2, a pressure module 11, an ultrasound module 12, an electrophoresis module 13, an electroporation module 14, an analysis module 20, a control module 21, and a measurement module 22. The connections between the modules are made by means of an inert piping 9. The inert piping 9 can also connect each module with each other, creating a bypass of the individual modules, the so-called bypass (not shown) being used in the event of a need to exclude a given module from the operation of the modular biotechnological installation. The installation may also contain two or more modules of the same type, which operate alternately, enabling servicing without a need to interrupt the operation of the installation. The measurement module 22, the analysis module 20 and the control module 21 are connected to each of the operating modules in order to, respectively, collect data, process data and send signals to the appropriate modules to control their operation. The indicated pumping system 3 is connected to all the modules in a manner obvious to the person skilled in the art, enabling flow of organic medium between the closed tank 1, and the remaining modules of the modular biotechnological installation.
   In addition, Figure 1 shows a renewable energy source (RES) module 17 and an energy module 16, which supply electricity to each of the remaining modules of the modular biotechnological installation.
Figure 2 shows a diagram of the construction of the modular biotechnological installation according to Example II, while Figure 3 shows a diagram of the construction of the modular biotechnological installation according to Example III.
Figure 4 shows the effect of electromagnetic field on algae production, wherein the X-axis shows the time of the experiment (days) and the Y-axis shows the amount of algae.
Figure 5 shows the effect of light on algae production, wherein the X-axis shows the time of the experiment (days) and the Y-axis shows the amount of algae.
Figure 6 shows the effect of pressure on algae production, wherein the X-axis shows the time of the experiment (days) and the Y-axis shows the amount of algae.
Figure 7 shows the effect of the feed (breeding) module 19 on algae production, wherein the X-axis shows the time of the experiment (days) and the Y-axis shows the amount of algae.
Figure 8 shows the effect of negative pressure on algae production, wherein the X-axis shows the time of the experiment (days) and the Y-axis shows the amount of algae.

### Example I

In one of the preferred embodiments of the invention, shown in Figure 1, the bioreactor comprises a closed tank 1 with a volume of 3 m³, wherein the tank 1 is made of a layered material comprising a layer of a polyester carrier with high tensile strength, wherein on each of the two sides of the polyester carrier there is, successively, an impregnating layer, a main PVC coating, a bonding primer layer and a topcoat layer. The Young's modulus of the elastic material is between 1.80 and 3.00 GN/m². For larger tanks, the Young's modulus can reach approx. 4.00 GM/m². It should be noted that the Young's modulus is temperature dependent. The closed tank 1 can operate under pressure. Alternatively, tank 1 can have a capacity of, for example, 10,000 L or 50,000 L or 100,000 L.

The polyester carrier with high tensile strength is a mesh constituting the main matrix with a honeycomb structure with a mesh size of up to 2 mm.

The impregnating layer is a hydrophobic coating containing PVC or PU (polyester urethane) or acrylic or polyurethane or silicone or DWR (Durable Water Repellency) or a combination thereof. In an example, an impregnating agent containing acetone (25-30% by weight), isopropyl acetate (1-3% by weight), C₈-C₁₂ hydrocarbons, n-alkanes, isoalkanes, cyclic compounds, aromatics (30-50% by weight, including aromatics 20-25% by weight), C₆-C₇ hydrocarbons, n-alkanes, cyclic isoalkanes, (25-30% by weight), including <5% n-hexane (Zeltimprägnierer (No. 07.1399.00 from Yachticon)) can be used.

The double-sided main coating made of the highest quality PVC can be applied by: direct coating, transfer coating, line coating, melt coating, coating of materials by the calendering method.

The primer layer contains a single-component resin or a multi-component resin adhesive that allows for the creation of a uniform bonding layer. The primer is a preparation that strengthens the substrate, improves its adhesion and reduces absorbency. Primer PVC - a single-component primer dedicated exclusively to the preparation of the surface of existing PVC membranes can be used.

The topcoat layer contains a two-component or multi-component varnish that allows for creation of a layer on the final surface that is mainly resistant to abrasion and UV radiation. For example, VERNIS ANTI-UV varnish (code: ST.CO-VUV) can be used.
The wall thickness of tank 1 is preferably between 3 and 5 mm.

The closed tank 1 is connected to a pumping system 3, comprising a salt/fresh water circulation pump, a measurement module 22, a feed module 19 for cultivating the feed biomass, a product purification module 7, a lighting module 15, and a dosing module 10.

The measurement module 22, comprising a set of sensors and meters, is configured to measure electrical energy [kWh or MWh], temperature [°C or °K], pressure [Pa or bar or psi], salinity [PSU or unitless], pH [0-14, unitless], certainty factor-CF [0-1, unitless], redox index-ORP [mv], heat coefficient-S.G. [W/mK], electrical conductivity-EC [mS], total content of mobile charged ions-TDS [µS/cm or ppm], turbidity of the medium [ntu or ebc], amount of biomass [kg or %], gas concentration [%]. The measurement module 22 provides data to the control module 21.

The product purification module 7 is a completely closed pressure filter. The product purification module 7 can be equipped with an integrated or external UV lamp, preferably with a capacity of at least 50 W. The product purification module 7 is responsible for purifying hydrogen from algae before further processing, ensuring high quality of the final product.

The lighting module 15 provides algae with light necessary for photosynthesis. It can use both natural and artificial light sources. The lighting module 15 contains a neon 5050 RGB LED strip, mounted in a waterproof cover enabling efficient and quick replacement or change of operating parameters. Diode type: 5050+WS2812B 3-chip RGB Epistar (each diode emits a separate light from the RGB palette, also in white). Tape length: 4 rolls of 5 m, width: 10 mm, number of diodes: 60 led/m, possibility of dimming and brightening the tape from 1 to 100%.

The dosing module 10 is designed to cyclically stabilize operation of the tank 1 by providing appropriate food and nutrient doses, providing algae with all the necessary substances for growth, and removing unnecessary old medium from the working volume. This is done by peristaltic pumps, which can work cyclically or continuously with set parameters and from transportable tanks containing an appropriate mixture (brine, food in the form of dissolved carbohydrates - cellulose and/or glucose and/or fructose and/or sucrose and dissolved nutrients - micro and macro elements), pumping the mixture into the working volume of tank 1, maintaining its operation. The dosing module 10 can also dose other substances, if necessary.

The pumping system 3 is a pumping system ensuring the circulation of liquid in tank 1 and the transport of nutrients and gases. It maintains appropriate conditions for the growth of algae. The pumping system 3 is connected to a separation module for algal biomass 23. The separation module for algal biomass 23 is connected to a purification module 4, which is connected to a sterilization module 6, which in turn is connected to a thermostatic module 5.

The separation module for algal biomass 23 is based on principles of buoyancy differences and flotation using carbon dioxide/oxygen. The separation module for algal biomass 23 may be in a form of a tank made of plastics, resistant to corrosion and chemicals (e.g. high density polyethylene - HDPE, polypropylene - PP, or polycarbonate). A cylindrical tank with a flat base was used, which ensures even accumulation of dead algae at the bottom. The lower and upper parts of the tank are narrowed to facilitate the collection of floating algae. The tank has the following dimensions - height: 1.5 m, diameter: 0.8 m, total capacity of about 750 liters.

The medium in the tank is sea water with algae (average density 1025 kg/m³). The flow velocity is 0.1 - 0.5 m/s. The average flow velocity is adjusted so as to avoid excessive turbulence that could disturb the separation.

The inlet is placed in the lower middle part of the tank and is directed vertically upwards, ensuring even distribution of the medium along the vertical axis of the tank while maintaining laminar flow conditions. The outlet is located in the upper central part of the tank, collecting alive algae that pass through the central zone, without floating or sinking.

Dead algae have a density lower or higher than seawater (around 900-1050 kg/m³), which causes them to float on the surface or sink to the bottom depending on the degree of degradation. Alive algae have a density close to seawater (1000-1020 kg/m³), which allows them to flow through the center of the tank without floating or sinking.

The tank is equipped with a CO₂/air dosing system, with a connection to a gas cylinder (or CO₂ supply system)/air pump, regulated by valves. Carbon dioxide/oxygen is introduced through micro-diffusers placed at the bottom of the tank, which disperse CO₂/O₂ in the form of microbubbles with a diameter of 10-50 micrometers, which attach to dead algae, lifting them to the surface. The concentration of CO₂/O₂ in the medium is precisely controlled to ensure effective flotation without negatively affecting alive algae. Typical levels are 0.5-1.5% of the volume of CO₂ in the tank. Additionally, due to the CO₂/air dosing system, the medium in the tank is aerated.

Dead algae, which have a greater tendency to sink, are collected at the bottom. The bottom of the tank is profiled in a cone shape, which facilitates the collection of material. A 50 mm diameter drain is placed at the lowest point of the conical bottom of the tank. The drain solenoid valve (e.g. ball) allows for periodic removal of dead algae from the bottom without interrupting the operation of the system.

Dead algae that float are collected on the surface. The upper part of the tank is equipped with a surface skimmer, which drains the floating algae to a separate tank.

The separation process is monitored by measuring the level of dissolved CO₂/O₂ in water using optical or chemical sensors. The automatic CO₂/O₂ dosing system ensures that the appropriate gas concentration is maintained in the water.

An optical system (e.g. refractometer) or ultrasonic system placed in the middle flow line monitors density and concentration of algae in the medium. The sensor controls the flow, ensuring optimal conditions for the separation of alive and dead algae.

The tank inlets and outlets are equipped with control valves that can be controlled automatically. The system can adjust the flow rate depending on the amount of algae and the degree of separation. The integrated system automatically removes dead algae from the lower and upper zones of the tank at regular intervals, without having to interrupt the operation. The micro-diffusers are automatically rinsed with fresh medium at high pressure to prevent them from being clogged by organic deposits. The tank is regularly rinsed using a pressure rinsing system equipped with cleaning nozzles placed in key places in the tank, which allows for regular removal of deposits without interrupting the separation process.

Additional equipment includes supply and discharge pipes with diameters of 20-50 mm, made of corrosion-resistant plastics, connected to a pumping system that regulates the flow of the medium. The entire system is electrically powered (power supply for pumps, sensors and control systems). Power consumption depends on the performance of the pumps and monitoring system, estimated at 2-5 kW for the entire system.

The separation module for algal biomass 23, based on differences in buoyancy and flotation by means of carbon dioxide/oxygen, works effectively due to the laminar flow of the medium, precise CO₂/O₂ dosing and an effective system for collecting dead algae from the lower and upper zones of the tank. The system is automated, which allows for continuous operation with minimal supervision, and regular cleaning and rinsing ensures operational reliability.

The purification module 4 is used to purify the organic medium from old microorganisms, which are removed from tank 1. The purification module 4 is also responsible for purifying water and nutrients before they are introduced into tank 1, which ensures better quality of algae cultivation. Purification module 4 is a separate tank, in which old microorganisms are removed by means of a brush and/or automatic robots. Alternatively, the purification module 4 can be located inside tank 1, wherein the removal of old microorganisms can take place by suction from the bottom of tank 1.

The sterilization module 6 sterilizes the tank 1 and the organic medium, preventing contamination by unwanted microorganisms. The sterilization module 6 contains a UV lamp with a power of at least 50 W.

The thermostatic module 5 maintains the optimum temperature in tank 1, which is crucial for efficient growth of algae, by creating a thermostatic circuit with a separate circulation pump on tank 1. The thermostatic module 5 is equipped with electric heating (thermostat with electric heating with a power of at least 3000 W, heating material: austenitic steel / metal block d) and a passive cooling system with a power of up to 11000 W. Alternatively, a Peltier cooling system with a power of 100 W per 1 m³ can be used. Thermostatic module 5 is connected to a gas exchange module 8, which is connected to a filtration-separation module 18.

The gas exchange module 8 controls carbon dioxide and oxygen levels in tank 1, which is crucial for photosynthesis and algae respiration. The gas exchange module 8 contains an air pump with a power of at least 80 W and a capacity of at least 5000 m³/h with an air diffuser. The gas exchange module ensures gas exchange of an air or enriched gas mixture containing CO₂ from outside and its delivery to tank 1. Gas exchange module 8 contains an external gas cylinder of 8 or 40 kg and a dedicated CO₂ diffuser.

Alternatively, the gas exchange module 8 may contain a dual zeolite system with a regenerable or replaceable cartridge powered by an air pump. Zeolite is designed to absorb nitrogen and oxygen to increase the concentration of CO₂ in the gas mixture, then in the next step they are automatically released into the atmosphere and the deposit is gradually regenerated for a new cycle.

The gas exchange module 8 may contain a foaming system containing nozzles with plastic meshes, causing the medium in the system to foam, which improves gas exchange by increasing the surface of contact between the medium and the gas mixture.

The filtration-separation module 18 filters and purifies the air entering the installation, providing optimal conditions for algae growth. The filtration-separation module 18 contains a pressure filter, preferably with a UV lamp, which are completely closed and can remain switched on continuously, because there is no risk of overflow. The filtration-separation module 18 ensures a stable biological balance. Special filter materials are used in the filtration process, e.g. yellow and blue sponges with different water flow rates having a large surface area, which in turn allows bacteria to settle.

Advantageously, the filtration-separation module 18 also includes a UV lamp, which supplements the filtration process by eliminating old and weak algae and parasites. In the exemplary embodiment, the UV lamp yield was 11 W. The pump pushes the water from the bottom of the tank 1 to the pressure filter, in which the water passes through all the filter media. At the end of the process, before it exits the filter, it is irradiated with light from the UV lamp. The water is then transported by means of a hose to the place where the leak is to occur. The filter advantageously includes an integrated cleaning system, which makes its operation much easier. It is sufficient to turn off the water supply to the filter and turn the crank on the head of the device, which is thereby cleaned. Then the direction of the waterflow must be changed by means of a switch so that it flows out through the outlet hose and the dirty water is rinsed out of the filter. A contamination indicator is provided on the head of the filter, which shows whether it is time to clean the filter media. In the exemplary embodiment, the recommended flow rate is up to 6000 L/h. Filtration volume 16 L. Dimensions of the filtration-separation module 18 - 310x310x450 mm.

The filtration-separation module 18 (like each module) has several tasks, e.g.: air filtration from solid impurities, supply of purified air to the organic medium and preliminary cleaning of the medium from dead algae.

The gas exchange module 8 is connected to an electromagnetic radiation module 2, which is connected to a pressure module 11, which is connected to an ultrasound module 12, which in turn is connected to a lighting module 15.

The electromagnetic radiation module 2 is used to stimulate the growth of algae and increase production efficiency. The electromagnetic radiation module 2 contains a power source, a 3000 kVA transformer allowing to generate high energy values for the conducted process, a tank for the medium with a magnetic coil placed around it, and an electromagnetic field meter.

Pressure module 11 enables a change of pressure in the cultivation process, which can affect the growth rate and efficiency of algae production. Pressure module 11 contains three alternating sections - two with increased pressure (up to 5 bar) and one with a slight negative pressure (-0.1 bar). Their task is to improve gas exchange of the cultivated product. Flows through the sections take place within the parameters set by the user.

An ultrasound module 12 uses ultrasounds to improve exchange of substances in tank 1, which can increase production efficiency. Ultrasound module 12 with ranges of 20, 40, 100 kHz is to generate low-power waves, inaudible to humans, due to the reverse piezoelectric effect, "opening" the cultivated cells and helping in the gas exchange of the working medium.

The ultrasound module 12 is connected to the electrophoresis module 13, the electroporation module 14 and the lighting module 15, the lighting module 15 being connected to tank 1.

The electroporation module 14 enables use of an electric field with very low voltages to reversibly damage cell membrane in order to transform the cells or their membranes to facilitate the process of escaping gaseous products and to facilitate the process of their nutrition. The electric field is used to introduce substances into the algae cells, which can support genetic modifications and increase production efficiency. Electroporation takes place on stainless steel grids in the built-in electroporation module 14.

Electrophoresis enables separation of different fractions of algal biomass using electric field, which is useful in purification and processing. The electrophoresis module 13 is constructed similarly to the electroporation module 14, with the difference that in the electrophoresis module 13 the organic medium circulation is temporarily closed so that the medium is not in motion, and the molecules themselves move to the +/- field that acts on them.

The feed module 19 for culturing the feed biomass, being a closed tank containing the feed biomass, is connected to an analysis module 20, and the analysis module 20 is connected to a control module 21. The control module 21 is connected to a measurement module 22, the feed module 19 for culturing the feed biomass and the closed tank 1. The data collected by the measurement module 22 are analyzed by the analysis module 20. The control module 21, which is connected to the analysis module 20 and measurement module 22, is configured to regulate the parameters: energy, temperature, pressure, salinity, pH, certainty factor-CF, redox index-ORP, heat coefficient-S.G., electrical conductivity-EC, total content of mobile charged ions-TDS, turbidity of the medium, amount of biological material, gas concentration. The feed module 19 is configured for growing the feed biomass intended to supply the organic medium of the closed tank 1.

The feed module 19 provides for conducting a closed process without adding substrates from outside the installation, increasing and ensuring the stability and efficiency of hydrogen production. Said feed module 19 provides self-sufficiency and no need to supply the organic medium with food from outside.

The connections between the modules are made by means of inert piping 9, which is a system of pipes made of chemically inert materials, transporting fluids and gases between the modules of the installation.

The modules of the biotechnological installation are powered by a renewable energy source (RES) module 17 and preferably additionally by an energy module 16. The energy module 16 may be in a form of an electrical switchboard. Preferably, the RES module 17 is used that uses renewable energy sources (e.g. solar panels, wind turbines) to power the installation, which reduces operating costs and environmental impact by reducing the carbon footprint. Modules 16 and 17 provide energy efficiency and self-sufficiency of the modular biotechnological installation without external energy interference, which reduces the carbon footprint.

In Fig. 1, the arrows indicate the flow directions of the organic medium (fluid connection). The dashed lines indicate electrical connections and the dotted lines - gas flow.

In one embodiment of the method according to the invention, the following steps are included:
- providing a modular biotechnological installation,
- supplying the organic medium, including microalgae together with a salt solution necessary for the life of the microalgae, known to a person skilled in the art, into a closed tank 1 and completely filling the closed tank 1 with the organic medium in such a way that there is no free space between the organic medium and the upper part of the material of the closed tank 1,
- supplying a gas mixture containing CO₂ to the closed tank 1, wherein the mixture containing CO₂ comes from a feed module 19 for culturing the feed biomass and is a mixture of gas products produced by the biomass cultivated in the feed module 19 for culturing the feed biomass, and the gas mixture is subjected to treatment by means of a filtration-separation module 18,
- converting the gas mixture into gas products, due to the fact that the gas mixture supplied to the closed tank 1 is converted by microalgae into a mixture of gas products, which in turn contains, among others, H₂ and CO₂,
- returning the concentrated gas mixture containing CO₂ to the closed tank 1, wherein it is not necessary to supply the gas mixture again and at the same time, in this embodiment, CO₂ is not a waste product that would be emitted into the atmosphere,
- supplying food for the organic medium to the closed tank 1, wherein in this embodiment, the food is the feed biomass cultivated in the feed module 19 for culturing the feed biomass and cellulose and nutrients originating from bamboo cultivation,
- removing excess of used organic medium from the closed tank 1 by scraping off the used organic medium with a scraper suitably adapted for this purpose and known to a person skilled in the art,
- supplying organic medium to the closed tank 1.
Optionally, food for the organic medium may be supplied from external sources.

Throughout the above steps of the method, the measurement module 22 measures the conditions prevailing in the installation, in particular the temperature, pressure, salt concentration, sugar content indicating the productivity of the microalgae being a component of the organic medium and transmits this data to the analysis module 20. In one embodiment, the analysis module 20 is integrated with software operating on the principle of artificial intelligence, which processes the input data obtained from the measurement module 22 and indicates which of the modules of the modular biotechnological installation should be switched on or off. In the present embodiment, in order to obtain the best possible efficiency of the production of the gas mixture, the organic medium flows through all the modules indicated in Fig. 1, which are switched on by the control module 21 on the basis of the parameters obtained by measurement by means of the measurement module 22 and processed by the analysis module 20. Thus, the organic medium from the closed tank 1 is moved by means of the pumping system 3, through the separation module for algal biomass 23, purification module 4, sterilization module 6, thermostatic module 5, gas exchange module 8, electromagnetic radiation module 2, pressure module 11, ultrasound module 12, electrophoresis module 13, electroporation module 14, lighting module 15. In the present embodiment, the gaseous product mixture is continuously recovered from the closed tank 1 and purified and separated into individual gas fractions by means of the product purification module 7.

In the present embodiment, the used organic matterfrom the separation module for algal biomass 23, purification module 4 and sterilization module 6 is led to further processes processing, so that it can be used, for example, as a fertilizer in agriculture.
In another preferred embodiment, the method is the same as described above, with the difference that the source of the gas mixture supplied to the closed tank 1 is atmospheric air.

The modular biotechnological installation according to example I for the production of algae contains the most advanced modules, which enable full control over the production processes and ensure the highest quality and efficiency. Thanks to the use of modern technologies, such as artificial intelligence, ultrasound, electroporation and renewable energy sources, the installation allows for the production of hydrogen from algae on a large scale with minimal impact on the environment. Each module plays a specific role, supporting various biological, mechanical and control processes, which enables comprehensive and efficient production.

### Example II

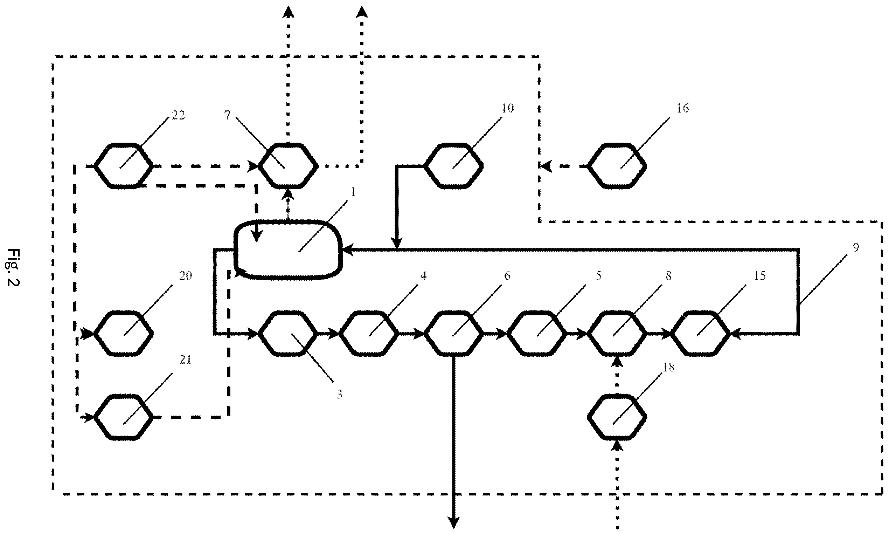

Figure 2 shows an alternative, simplified construction of a biotechnological installation. In this variant, as compared to the installation of Example 1, the installation does not have an electromagnetic radiation module 2, a pressure module 11, an ultrasound module 12, an electrophoresis module 13, an electroporation module 14, a RES module 17, a feed module 19 for feed biomass cultivation and a separation module for algal biomass 23.

The modular biotechnological installation according to Example II for the production of algae contains modules that improve the control and quality of production. Thanks to the purification module 4, the filtration module 18 and the analysis module 20, the installation provides better conditions for algae growth and higher quality of the final product. Each module plays a specific role, supporting various biological, mechanical and control processes, which enables more advanced and efficient production of algae.

### Example III

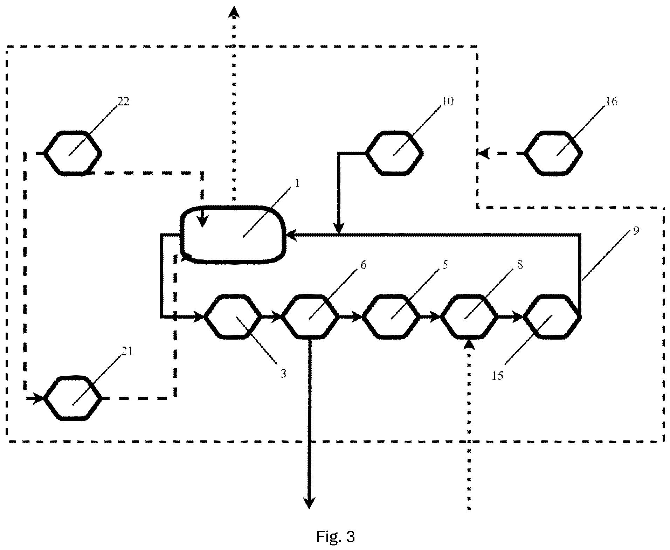

Figure 3 shows an alternative, simplified construction of a biotechnological installation. In this variant, in comparison with the installation of Example 1, the installation does not have an electromagnetic radiation module 2, a purification module 4, a gaseous product purification module 7, a pressure module 11, an ultrasound module 12, an electrophoresis module 13, an electroporation module 14, a RES module 17, a filtration-separation module 18, a feed module 19 for feed biomass cultivation, an analysis module 20 and a separation module for algal biomass 23.

The modular biotechnological installation according to Example III for the production of algae consists of key modules that provide the basic functions necessary for effective algae cultivation. Each module plays a specific role, supporting biological, mechanical and control processes, which enables stable production of unpurified hydrogen.

### Example IV

The modular biotechnological installation according to Example 1 was used for algae cultivation with the application of magnetic field, light and pressure. The lighting module 15 contained a neon 5050 RGB LED strip, mounted in a silicone waterproof cover enabling its efficient and quick replacement or change of operating parameters, mainly the color range, power and its operating times in cycles in order to increase the efficiency of the bioreactor.

Pressure module 11 contained three alternating sections - two with increased pressure (up to 5 bar) and one with a gentle negative pressure (-0.1 bar). Their task was to improve the gas exchange of the cultivated product.

Electromagnetic radiation module 2, due to the phenomenon of magnetic field induction in a twisted wire, generated an electric and magnetic field, which in the category of their superposition is known as an electromagnetic field. Electromagnetic field of very low frequency (1-80 Hz) has a positive effect on the body, causing acceleration of enzymatic processes, faster regeneration of damaged cells and a change in their structure (e.g. increased tissue density). Many sources confirm that under the influence of such a field, positive changes in the activity of some enzymes and biochemical processes can be observed, to which the above module is adapted, consisting of:
- a power source
- a 3000 kVA transformer allowing to generate significantly higher energy values for the conducted process
- a tank for the medium with a magnetic coil placed around it
- an attached electromagnetic field meter for its verification

The influence of the above parameters selected in appropriate cycles increased the efficiency of the entire biotechnological installation.

Table 1 presents the results of the 19-day experiment under the conditions given in Table 1.
- Basic statistics:
   Average amount of algae / production: The average value of algae production in the sample data is about 10.9.
   Median: The median, or middle value, is about 11.2.
   Standard deviation: A measure of variability of the algae production value around the average value is about 3.33.
- Observations:
   The lowest algae production value is 3.2 and the highest is 15.6.

Most algae production values are between 8.6 and 14.3.

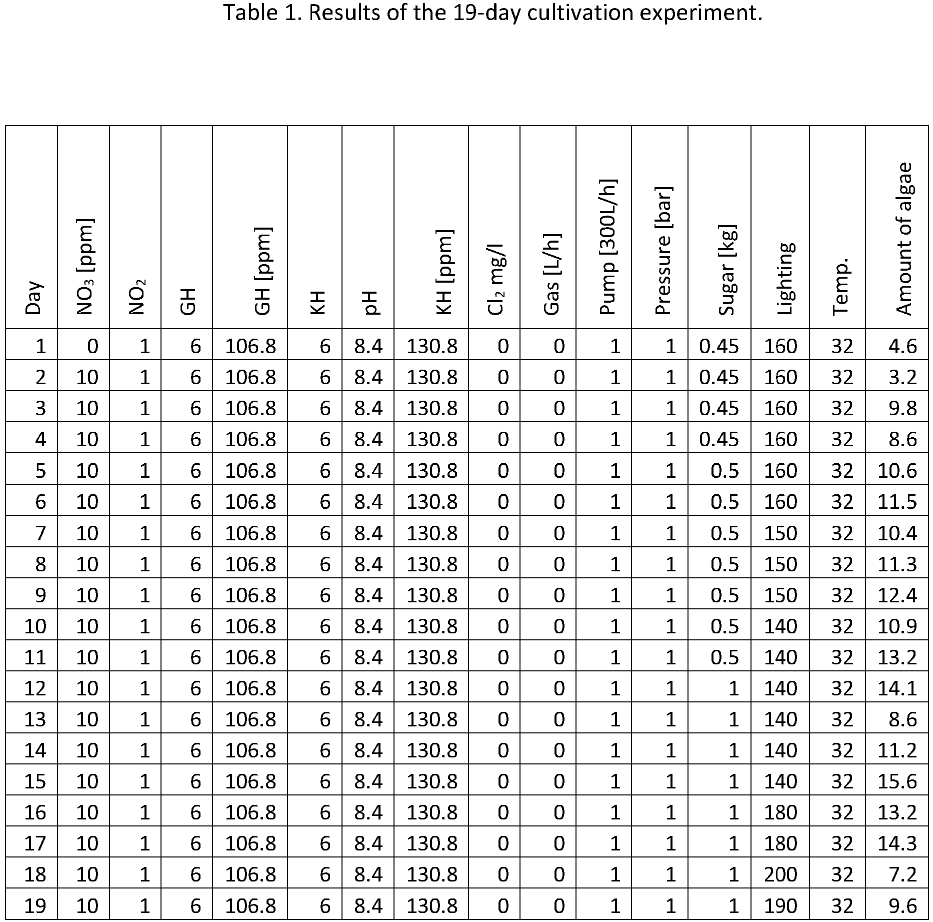

Production values are quite diverse, which is caused by the influence of various experimental factors on production and the process itself related to the cyclical nature of the process.

The unit of algae amount was the mass of algae in kg per volume of 1000 L after drying to a stable substance.

### Influence of the electromagnetic field

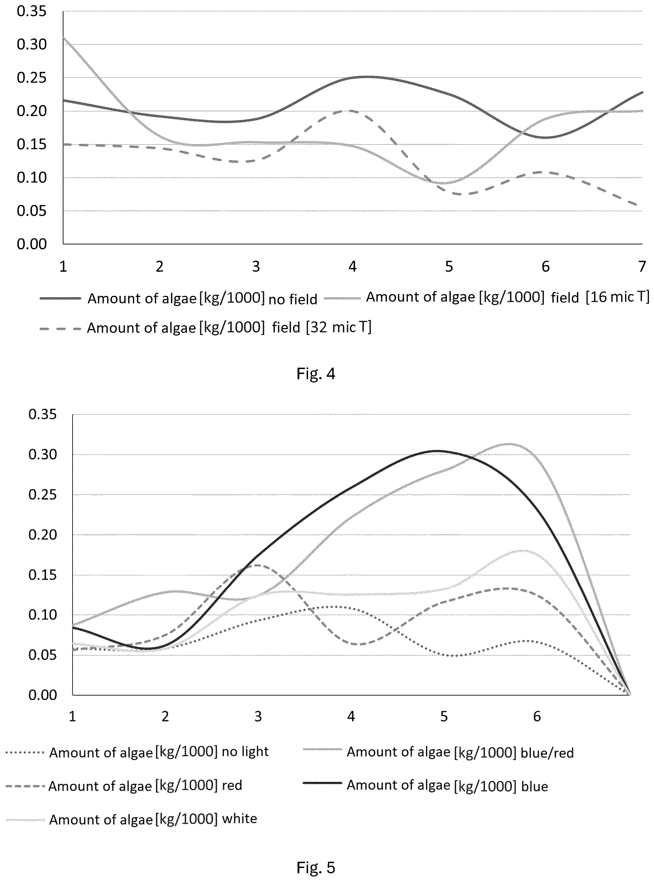

A 7-day experiment was carried out using the electromagnetic radiation module 2. Its influence is presented in Table 2 and in Figure 4, where the X-axis shows the time of the experiment (days), while the Y-axis shows the amount of algae.

**Table 2. Influence of the electromagnetic radiation module 2 on algae production.**

| Day | Amount of algae [kg/1000] No field | Amount of algae [kg/1000] field [16 micT] | Amount of algae [kg/1000] field [32 micT] |
|---|---|---|---|
| 1 | 0.22 | 0.31 | 0.15 |
| 2 | 0.19 | 0.16 | 0.14 |
| 3 | 0.19 | 0.15 | 0.13 |
| 4 | 0.25 | 0.15 | 0.20 |
| 5 | 0.23 | 0.09 | 0.08 |
| 6 | 0.16 | 0.19 | 0.11 |
| 7 | 0.23 | 0.20 | 0.06 |

**Table 3. Statistics for data from Table 2**

| Statistics | No field | Field [16 micT] | Field [32 micT] |
|---|---|---|---|
| Average | 0.21 | 0.18 | 0.13 |
| Median | 0.22 | 0.16 | 0.13 |
| Standard deviation | 0.03 | 0.07 | 0.04 |
| Minimal value | 0.16 | 0.09 | 0.06 |
| Maximal value | 0.25 | 0.31 | 0.20 |

### Observations:

Without magnetic field: The average amount of algae produced is 0.21 kg/1000, with a standard deviation of 0.03. The lowest value is 0.16 kg/1000 and the highest is 0.25 kg/1000.
Magnetic field [16 µT]: The average amount of algae produced is 0.18 kg/1000,
with a standard deviation of 0.07. The lowest value is 0.09 kg/1000 and the highest is 0.31 kg/1000.
Magnetic field [32 µT]: The average amount of algae produced is 0.13 kg/1000,
with a standard deviation of 0.04. The lowest value is 0.06 kg/1000 and the highest is 0.20 kg/1000.

### Conclusions:

Influence of magnetic field: The data shows that algae production is highest without the presence of magnetic field (average 0.21 kg/1000). Algae production decreases with increasing magnetic field strength, reaching its lowest values at 32 microtesla (average 0.13 kg/1000).
Reduction in production: The presence of a magnetic field of 32 microtesla seems to have the greatest negative effect on algae production.
Variance: The larger standard deviation for a field of 16 microtesla suggests greater variability of the results for this case as compared to other conditions.

### Interpretation of results:

The use of the electromagnetic radiation module negatively affected algae production by selecting the strongest individuals. According to literature reports, the effect of electromagnetic radiation on plant and animal populations to select only the strongest individuals is based on the effect on the biological processes of organisms. The literature describes a variety of ways in which low-level electromagnetic fields can affect various plant and animal species (Levitt BB, Lai HC, and Manville AM II (2022) Low-level EMF effects on wildlife and plants: What research tells us about an ecosystem approach, Front. Public Health 10:1000840; Levitt BB, Lai HC, and Manville AM II (2022) Effects of non-ionizing electromagnetic fields on flora and fauna, Part 2 impacts: how species interact with natural and man-made EMF, Front. Rev Environ Health 2022; 37(3): 327-406; https://www.bfs.de/EN/bfs/science-research/emf/statements/emf-animals-and-plants.html).

Controlling populations to obtain the "strongest" individuals may involve:
- UV radiation or other ionizing radiation, which induces mutations in DNA, leading to natural selection for the strongest individuals that survive the mutations and adapt.
- Controlling plant growth using visible light - changing the intensity and wavelength of light affects photosynthesis, flowering and metabolism of plants. Under the right conditions, the development of the strongest plants can be accelerated.
- High-frequency electromagnetic fields affect all biological elements. This may cause only individuals with stronger adaptive mechanisms to survive.

### Influence of light

A 6-day experiment was conducted using the lighting module 15 using blue, red and white light. Its influence is shown in Table 4 and in Figure 5, where the X-axis shows the time of the experiment (days), while the Y-axis shows the amount of algae.

**Table 4. Influence of lighting module on algae production**

| Day | Amount of algae [kg/1000] no light | Amount of algae [kg/1000] blue/red | Amount of algae [kg/1000] red | Amount of algae [kg/1000] blue | Amount of algae [kg/1000] white |
|---|---|---|---|---|---|
| 1 | 0.06 | 0.09 | 0.06 | 0.08 | 0.06 |
| 2 | 0.06 | 0.13 | 0.08 | 0.06 | 0.06 |
| 3 | 0.09 | 0.12 | 0.16 | 0.18 | 0.12 |
| 4 | 0.11 | 0.22 | 0.06 | 0.26 | 0.13 |
| 5 | 0.05 | 0.28 | 0.12 | 0.30 | 0.13 |
| 6 | 0.07 | 0.29 | 0.12 | 0.23 | 0.18 |

### Descriptive statistics:

- Algae amount [kg/1000] no light
   Average: (0.06 + 0.06 + 0.09 + 0.11 + 0.05 + 0.07) / 6 = 0.0733
   Median: 0.065, Minimum: 0.05, Maximum: 0.11, Standard deviation: 0.0214
- Algae amount [kg/1000] blue/red
   Average: (0.09 + 0.13 + 0.12 + 0.22 + 0.28 + 0.29) / 6 = 0.1883
   Median: 0.175, Minimum: 0.09, Maximum: 0.29, Standard deviation: 0.0783
- Algae amount [kg/1000] red
   Average: (0.06 + 0.08 + 0.16 + 0.06 + 0.12 + 0.12) / 6 = 0.10
   Median: 0.1, Minimum: 0.06, Maximum: 0.16, Standard deviation: 0.0396
- Algae amount [kg/1000] blue
   Average: (0.08 + 0.06 + 0.18 + 0.26 + 0.30 + 0.23) / 6 = 0.185
   Median: 0.205, Minimum: 0.06, Maximum: 0.30, Standard deviation: 0.0913
- Algae amount [kg/1000] white
   Average: (0.06 + 0.06 + 0.12 + 0.13 + 0.13 + 0.18) / 6 = 0.1133
   Median: 0.125, Minimum: 0.06, Maximum: 0.18, Standard deviation: 0.0444

### Conclusions:

Algae [kg/1000] no light: The average is 0.0733 kg/1000, which is the lowest average compared to other categories. This is a relatively stable category with a low standard deviation (0.0214).

Algae [kg/1000] blue/red: This category has the highest average (0.1883 kg/1000) and also the highest standard deviation (0.0783), indicating a greater variability in the amount of algae in this category.

Algae [kg/1000] red: The average amount of algae in this category is 0.10 kg/1000, which is relatively low, but higher than the "no light" category. The standard deviation is 0.0396, indicating moderate variability.

Algae amount [kg/1000] blue: The average (0.185 kg/1000) is very close to the "blue/red" category, but the variability is higher (standard deviation 0.0913).

Algae amount [kg/1000] white: The average algae amount is 0.1133 kg/1000 with a standard deviation of 0.0444. This is a relatively stable category compared to "blue/red" and "blue".

### Influence of pressure

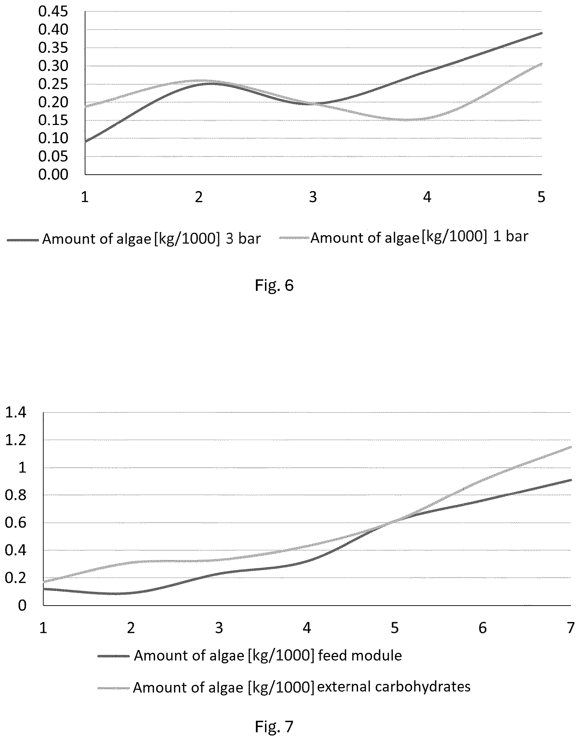

A 5-day experiment was conducted using pressure module 11. Its influence is shown in Table 5 and in Figure 6, where the X-axis shows the experimental time (days), while the Y-axis shows the algae amount.

**Table 5. Influence of pressure module 11 on algae production.**

| Day | Amount of algae [kg/1000] 3 bar | Amount of algae [kg/1000] 1 bar |
|---|---|---|
| 1 | 0.09 | 0.19 |
| 2 | 0.25 | 0.26 |
| 3 | 0.20 | 0.20 |
| 4 | 0.29 | 0.16 |
| 5 | 0.39 | 0.31 |

### Descriptive statistics:

- Algae amount [kg/1000] 3 bar
   Average: (0.09 + 0.25 + 0.20 + 0.29 + 0.39) / 5 = 0.244 kg/1000
   Median: 0.25 kg/1000, Minimum: 0.09 kg/1000, Maximum: 0.39 kg/1000, Standard deviation: 0.111 kg/1000
- Algae amount [kg/1000] 1 bar
   Average: (0.19 + 0.26 + 0.20 + 0.16 + 0.31) / 5 = 0.224 kg/1000
   Median: 0.20 kg/1000, Minimum: 0.16 kg/1000, Maximum: 0.31 kg/1000, Standard deviation: 0.058 kg/1000

### Conclusions:

Average algae amount: The "3 bar" category has a higher average algae amount (0.244 kg/1000) compared to the "1 bar" category (0.224 kg/1000).
Median algae amount: The median for "3 bar" is 0.25 kg/1000, which is higher than the median for "1 bar", which is 0.20 kg/1000.
Range of values: The algae amount in the "3 bar" category ranges from 0.09 to 0.39 kg/1000, which is a larger variability than in the "1 bar" category, which ranges from 0.16 to 0.31 kg/1000. Standard deviation: The standard deviation for "3 bar" is 0.111 kg/1000, indicating a greater variability in this category compared to "1 bar", which has a standard deviation of 0.058 kg/1000.

### Summary

The "3 bar" category is characterized by a higher average algae amount and a greater variability compared to the "1 bar" category. The median and range of values are also higher for "3 bar", suggesting that the algae amount in this category is more variable. The "1 bar" category is more stable, with a smaller standard deviation and a smaller range of values.

### Effect of the feed module

A 7-day experiment was conducted using the feed module 19 (also called the breeding module). Its effect is shown in Table 6 and in Figure 7, where the X-axis shows the experimental time (days) and the Y-axis shows the algae amount.

**Table 6. Effect of feed module 19 on algae production**

| Dzień | Amount of algae [kg/1000] feed module | Amount of algae [kg/1000] external carbohydrates |
|---|---|---|
| 1 | 0.12 | 0.17 |
| 2 | 0.09 | 0.31 |
| 3 | 0.23 | 0.33 |
| 4 | 0.32 | 0.43 |
| 5 | 0.61 | 0.61 |
| 6 | 0.76 | 0.91 |
| 7 | 0.91 | 1.15 |

### Descriptive statistics:

- Algae amount [kg/1000] feed module
   Average: (0.12 + 0.09 + 0.23 + 0.32 + 0.61 + 0.76 + 0.91) / 7 = 0.359 kg/1000
   Median: 0.32 kg/1000, Minimum: 0.09 kg/1000, Maximum: 0.91 kg/1000, Standard deviation: 0.266 kg/1000
- Algae amount [kg/1000] external hydrocarbons
   Average: (0.17 + 0.31 + 0.33 + 0.43 + 0.61 + 0.91 + 1.15) / 7 = 0.473 kg/1000
   Median: 0.43 kg/1000, Minimum: 0.17 kg/1000, Maximum: 1.15 kg/1000, Standard deviation: 0.301 kg/1000

### Conclusions:

Average algae amount: The "external hydrocarbons" category has a higher average algae amount (0.473 kg/1000) compared to "feed module" (0.359 kg/1000).
Median algae amount: The median for "external hydrocarbons" is 0.43 kg/1000, which is higher than the median for "feed module" (0.32 kg/1000).
Range of values: The amount of algae in the "external hydrocarbons" category ranges from 0.17 to 1.15 kg/1000, which is a larger range of values than in the "feed module" category, which ranges from 0.09 to 0.91 kg/1000.

### Standard deviation:

The standard deviation for "external hydrocarbons" is 0.301 kg/1000, indicating a greater variability in this category compared to "feed module", which has a standard deviation of 0.266 kg/1000.

### Summary:

The "external hydrocarbons" category has a higher average amount of algae and a greater variability compared to the "feed module" category. The median and range of values are also higher for "external hydrocarbons", suggesting that the amount of algae in this category is more variable. The "feed module" category is less variable, but still shows significant differences in algae amounts as compared to "external hydrocarbons".

### Effect of negative pressure

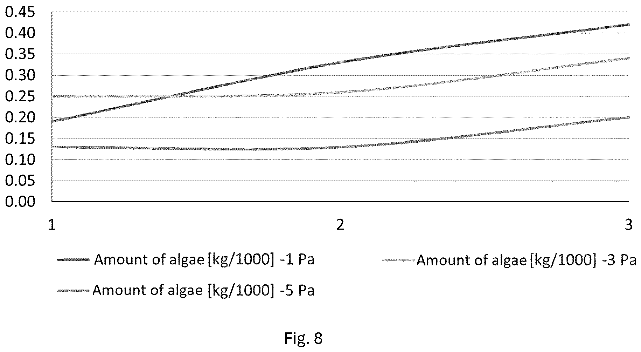

A 3-day experiment was conducted using the pressure module 11 to investigate the effect of negative pressure on algae production. Its effect is shown in Table 7 and in Figure 8, where the X-axis shows the experimental time (days) and the Y-axis shows the amount of algae.

**Table 7. Effect of negative pressure on algae production**

| Day | Amount of algae [kg/1000] -1 Pa | Amount of algae [kg/1000] -3 Pa | Amount of algae [kg/1000] -5 Pa |
|---|---|---|---|
| 1 | 0.19 | 0.25 | 0.13 |
| 2 | 0.33 | 0.26 | 0.13 |
| 3 | 0.42 | 0.34 | 0.20 |

### Descriptive statistics:

- Algae amount [kg/1000] -1 Pa
   Average: (0.19 + 0.33 + 0.42) / 3 = 0.31 kg/1000
   Median: 0.33 kg/1000, Minimum: 0.19 kg/1000, Maximum: 0.42 kg/1000, Standard deviation: 0.115 kg/1000
- Algae amount [kg/1000] -3 Pa
   Average: (0.25 + 0.26 + 0.34) / 3 = 0.283 kg/1000
   Median: 0.26 kg/1000, Minimum: 0.25 kg/1000, Maximum: 0.34 kg/1000, Standard deviation: 0.045 kg/1000
- Algae amount [kg/1000] -5 Pa
   Average: (0.13 + 0.13 + 0.20) / 3 = 0.153 kg/1000
   Median: 0.13 kg/1000, Minimum: 0.13 kg/1000, Maximum: 0.20 kg/1000, Standard deviation: 0.035 kg/1000

### Conclusions:

Average algae amount: The "-1 Pa" category has the highest average algae amount (0.31 kg/1000), while the "-5 Pa" category has the lowest average algae amount (0.153 kg/1000).

Median algae amount: The median for "-1 Pa" is 0.33 kg/1000, which is higher than the median for "-3 Pa" (0.26 kg/1000) and "-5 Pa" (0.13 kg/1000).

Range of values: The amount of algae in the "-1 Pa" category ranges from 0.19 to 0.42 kg/1000. The amount of algae in the "-3 Pa" category ranges from 0.25 to 0.34 kg/1000.

The amount of algae in the "-5 Pa" category ranges from 0.13 to 0.20 kg/1000.

The "-1 Pa" category has the widest range of values, while "-5 Pa" has the narrowest range. Standard deviation: The standard deviation for "-1 Pa" is 0.115 kg/1000, indicating the greatest variability in this category.

The "-3 Pa" category has a standard deviation of 0.045 kg/1000, suggesting moderate variability. The "-5 Pa" category has the smallest standard deviation (0.035 kg/1000), suggesting the lowest variability.

### Summary:

The "-1 Pa" category has the highest average algae amount and the greatest variability, with the widest range of values. The "-3 Pa" category has a moderate average algae amount and variability.

The "-5 Pa" category has the lowest average algae amount and the lowest variability, with a narrow range of values. This suggests that higher pressures are associated with more algae amount and greater variability in algae amount.

## Claims

1. A closed liquid bioreactor configured to operate under pressure, **characterized in that** it is in a form of a tank (1) made of an elastic material with a modulus of elasticity ranging from 0.02 to 9.5 GN/m², preferably between 1.8 and 4.0 GN/m², wherein the flexible material preferably comprises polyvinyl chloride or polyester or nylon.

2. The closed liquid bioreactor according to claim 1, **characterized in that** the flexible material has a layered structure and comprises a polyester carrier layer, wherein on each of the two sides of the polyester carrier there are successively:
- an impregnating layer,
- a main coating, preferably comprising PVC or polyester or polypropylene,
- a binding primer layer,
- a topcoat layer.

3. The closed liquid bioreactor according to claim 1, **characterized in that** the outlet of the tank (1) is made of a material limiting emission of gaseous products, comprising at least two matrices, wherein the material preferably comprises a main jacket and a collar, wherein the collar is made of the same or a different material than the material from which the jacket is made.

4. A modular biotechnological installation for culturing microorganisms, **characterized in that** it comprises:
- a closed liquid bioreactor adapted to operate under pressure according to claim 1 or 2 or 3, and
- a pumping system (3) configured to allow the flow of an organic medium containing bioorganisms and/or algal biomass between the closed tank (1) and remaining modules of the modular biotechnological installation,
- a thermostatic module (5) configured to increase, decrease or maintain a set temperature of the organic medium in the tank (1),
- a sterilization module (6) configured to sterilize the organic medium,
- a gas exchange module (8) configured to supply oxygen and/or CO₂ to the closed tank (1),
- inert piping (9) configured to connect the tank (1), the modules and the pumping system (3) in the installation,
- a dosing module (10) configured to supply food/nutrients to the organic medium,
- a lighting module (15) configured to regulate light and dark cycle for growth of bioorganisms and/or algal biomass,
- an energy module (16) configured to provide electrical energy for operation of the modules of the installation,
- a measurement module (22) configured to measure at least one of the parameters selected from: energy, temperature, pressure, salinity, pH, certainty factor-CF, redox index-ORP, thermal coefficient-S.G., electrical conductivity-EC, total content of mobile charged ions-TDS, turbidity of the organic medium, amount of biomass, gas concentration and to provide the measurement data to a control module (21), wherein the control module (21) is configured to control the operation of the biotechnological installation.

5. The modular biotechnological installation according to claim 4, **characterized in that** it comprises a purification module (4) configured to remove excess organic medium from the closed tank (1) and/or a gaseous product purification module (7), preferably in a form of a filter system and/or a filtration-separation module (18) for purifying air taken from the atmosphere and/or an analysis module (20) for analyzing measurement data from the measurement module (22), wherein the installation preferably comprises all modules (4, 7, 18, 20) at the same time.

6. The modular biotechnological installation according to claim 5, **characterized in that** it comprises an electromagnetic radiation module (2) configured to generate a low frequency electromagnetic field during the flow of the organic medium, and/or a pressure module (11) configured to increase or decrease pressure during the flow of the organic medium, and/or an ultrasound module (12) configured to emit ultrasounds in the organic medium, and/or an electrophoresis module (13) configured to apply voltage to the organic medium to separate different fractions of the organic medium, and/or an electroporation module (14) configured to damage cell membrane of the cells, and/or a renewable energy source module (17), and/or a feed module (19) for culturing bioorganisms or a feed biomass intended to feed the organic medium of the closed tank (1), and/or a separation module for bioorganisms and/or algal biomass (23), wherein the installation preferably comprises all of the modules (2, 11, 12, 13, 14, 17, 19, 23).

7. A method of production of gases using a modular biotechnological installation, **characterized in that** it comprises the following steps:
- providing a biotechnological installation as defined in any one of claims 4 to 6,
- supplying an organic medium containing bioorganisms and/or algal biomass to a closed tank (1) and partially or completely, preferably completely, filling the closed tank (1) with the organic medium,
- supplying a gas mixture to the closed tank (1),
- converting the gas mixture into gaseous products,
- returning a part of the concentrated mixture of gaseous products obtained in the previous step, containing CO₂ to the closed tank (1),
- supplying food for the organic medium to the closed tank (1),
- removing excess of used organic medium from the closed tank (1),
- re-supplying the organic medium to the closed tank (1),
wherein during performance of the method, the recovery of the gaseous product from the closed tank (1) is carried out and measurement of operating parameters of the installation and transfer of data to the control module (21) is carried out.

8. The method according to claim 7, **characterized in that** the steps of the method are carried out continuously or periodically depending on the conditions in the biotechnological installation measured by means of the measurement module (22).

9. The method according to claim 7 or 8, **characterized in that** the gas mixture in the step of supplying the gas mixture to the closed tank (1) is a mixture containing CO₂.

10. The method according to claim 7 or 8 or 9, **characterized in that** the gas product is a mixture containing hydrogen, nitrogen, oxygen and CO₂, which is then separated into fractions of individual gases.

11. The method according to one of claims 7 to 10, **characterized in that** the step of supplying food for the organic medium takes place via the feed module (19) for the feed biomass cultivation.

12. The method according to one of claims 7 to 11, **characterized in that** the installation according to claim 4 or 5 or 6 is used to carry out the method, wherein at least one module is operated periodically during the performance of the method.

13. The method according to one of claims 7 to 11, **characterized in that** the installation according to claim 4 or 5 or 6 is used to carry out the method, wherein at least one module is operated continuously during the performance of the method.
